# EUROPEAN PATENT SPECIFICATION

(11) **EP 2 459 532 B1**
(45) Date of publication and mention of the grant of the patent: **09.04.2014**
(21) Application number: 10739347.2
(22) Date of filing: 28.07.2010
(51) Int. Cl.: C07D 207/33, A61K 31/402, A61P 35/00

(54) **Antitumor 1,2-diphenylpyrrole compounds and their preparation process**
Antitumorigene 1,2-Diphenylpyrrolverbindungen und deren Herstellungsverfahren
Composés anti-tumoraux à base de 1,2-diphénylpyrrole et leur procédé de préparation

(30) Priority: 29.07.2009 EP 09166668; 29.07.2009 US 229643 P
(43) Date of publication of application: 06.06.2012
(73) Proprietor: Argon Pharma S.L., 08028 Barcelona (ES)
(72) Inventor: MANGUES BAFALLUY, Ramón, E-08005 Barcelona (ES); CASANOVA RIGALT, Isolda, E-08041 Barcelona (ES); ALBERICIO PALOMERA, Fernando, E-08028 Barcelona (ES); ÁLVAREZ, Mercedes, E-08970 Sant Joan Despí (Barcelona) (ES); SAVINA, Svetlana, E-08014 Barcelona (ES)
(74) Representative: ZBM Patents - Zea, Barlocci & Markvardsen
(86) International application number: PCT/EP2010/060983
(87) International publication number: WO 2011/012660

(56) References cited:
- EP-A- 0 927 555
- US-A- 5 908 858
- VACHAL PETR ET AL: "General facile synthesis of 2,5-diarylheteropentalenes" TETRAHEDRON LETTERS, vol. 45, no. 38, 13 September 2004 (2004-09-13), pages 7157-7161, XP002550237 ISSN: 0040-4039

## Description

This application claims the benefit of priority of European patent application EP 09166668 and U.S. Provisional Patent Application US 61/229,643, both filed on July 29, 2009.

The present invention relates to a new series of 1,2-diphenylpyrroles having antitumor activity, to a process for preparing them, and to pharmaceutical compositions containing them.

### BACKGROUND ART

Cancer is a heterogeneous disease characterized by the accumulation of tumor cells, which can cause the death of both animals and humans. Conventional methods of treating cancer include surgical treatments, the administration of genotoxic agents, and recently the administration of small molecule inhibitors of target receptors or the administration of an antibody or antibody fragments, which may be conjugated to a therapeutic moiety, targeting membrane receptors. However, to date, such treatments have been of limited success.

The development of antitumoral therapies of general applicability is one of the main goals in medicinal chemistry. Among the strategies envisaged for developing new antitumoral treatments, the promotion of cell death, in particular the induction of apoptosis by the inhibition of signaling through focal adhesions, a mechanism barely explored so far, is especially attractive. The reason for this interest lies in the antitumor and antimetastatic activity induced by the blockade of the constitutive signaling through focal adhesions. Constitutive signaling through focal adhesions occurs in most if not all neoplastic, when they become transformed and acquire the capacity for anchorage independent growth, a process that participates in tumor growth and metastatic spread. In this context, the development of new compounds which can act as apoptotic inductors in as many cancers as possible would have an enormous scientific, social and economic impact.

Although many drugs have been used in cancer therapy, at present there is no curative therapy for most types of cancer.

EP 927555 describes the use of 1,2-diphenylpyrrole derivatives for the treatment or prevention of tumours, tumour-related disorders and cachexia. The compounds of this application are COX-2 inhibitors. Compounds showing selective COX-2 inhibition were initially developed for its use as antiinflammatory drugs, and later developed as antitumor agents. However, it has been described that COX-2 inhibitors may exhibit some undesired effects, such as cardiovascular toxicity.

Therefore, there is still an interest in developing compounds which show improved activity in the treatment of cancer and less undesired effects.

### SUMMARY OF THE INVENTION

Inventors have found a new series of 1,2-diphenylpyrroles that shows an enhanced antitumor activity. In particular, the 1,2-diphenylpyrroles of the invention, which comprise two substituents on the phenyl ring attached at the 2-position of the pyrrole ring, show a significant lower tumor cell viability in cancer, wherein the cancer is selected from the group consisting of lung carcinoma, colorectal carcinoma, breast carcinoma and prostate carcinoma.

Therefore, a first aspect of the present invention refers to compounds of formula (I), or their pharmaceutically acceptable salts or their pharmaceutically acceptable solvates, including hydrates 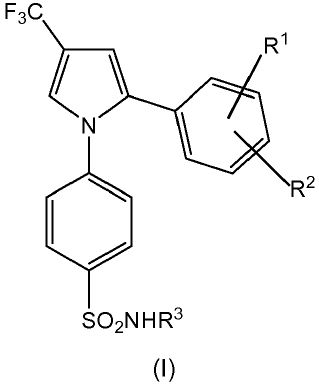 wherein R¹ and R² are individually selected from halogen, O(C₁-C₄)alkyl optionally substituted with one or more halogen atoms, and (C₁-C₄)alkyl optionally substituted with one or more substituents selected from halogen, OH and O(C₁-C₄)alkyl; and R³ is selected from H, (C₁-C₄)alkyl, CONH₂ and (C=NH)NH₂.

Another aspect of the present invention relates to a process for the preparation of the compounds of formula (I) as defined above, which comprises:
a) reacting a compound of formula (II) with a compound of formula (III) 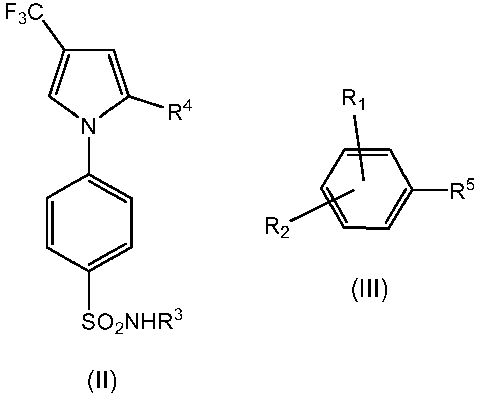 in the presence of a metal catalyst and a base, wherein R¹, R² and R³ have the same meaning as defined above; R⁴ represents X or Y; R⁵ represents X when R⁴ represents Y, or R⁵ represents Y when R⁴ represents X; X is selected from halogen, trifluoromethanesulfonate and R₃SiO; Y is selected from SnR₃, ZZnMg, ZZnCu, B(OH)₂, B(OR)₂ and 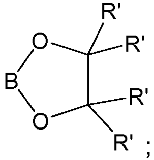 R represents (C₁-C₄)alkyl; Z represents halogen; and R' represents H or (C₁-C₄)alkyl;
b) optionally converting, in one or a plurality of steps, a compound of formula (I) into another compound of formula (I); and
c) optionally reacting a compound of formula (I) with a base or an acid to give the corresponding salt.

Another aspect of the present invention relates to a pharmaceutical composition which comprises an effective amount of a compound of formula (I) as defined above, together with one or more pharmaceutically acceptable excipients or carriers.

As previously described, the compounds of the invention are useful for the treatment of cancer. Therefore, another aspect of the present invention relates to a compound of formula (I) as defined above, for the treatment of cancer. Thus, this aspect relates to the use of a compound of formula (I) as defined above, for the manufacture of a medicament for the treatment of cancer; and may also be formulated as a method for the treatment of cancer comprising administering an effective amount of the previously defined compound of formula (I), and one or more pharmaceutical acceptable excipients or carriers, in a subject in need thereof.

These aspects of the present invention will be further described in the detailed description section that follows. Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional aspects, advantages and features of the invention will become apparent to those skilled in the art upon examination of the description or may be learned by practice of the invention. Furthermore, the present invention covers all possible combinations of particular and preferred embodiments described herein.

### BRIEF DESCRIPTION OF THE DRAWINGS

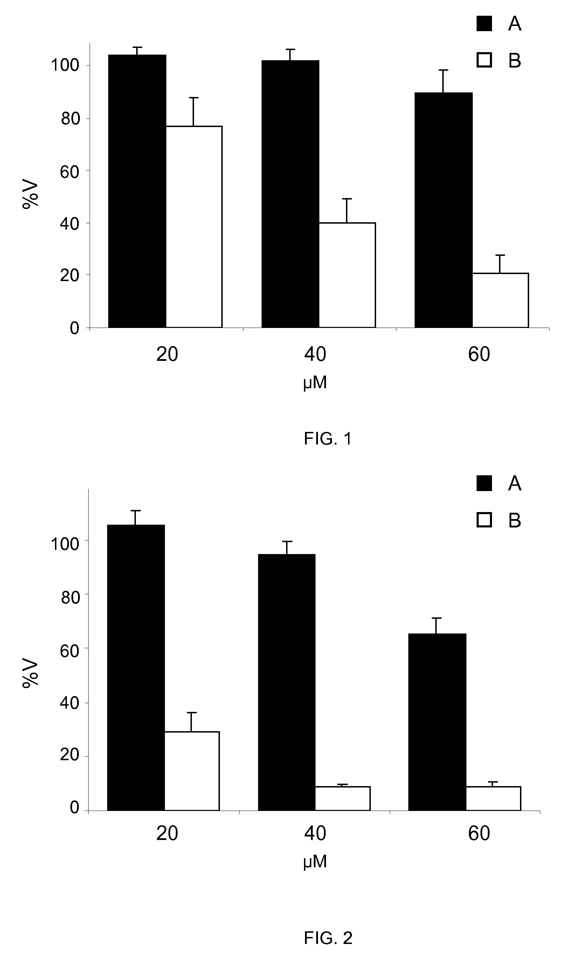

FIG. 1 shows the tumor cell viability after exposing the LS174T cell line (derived from human colorectal carcinoma cells) during 4 hours to 20, 40 or 60µM concentrations of compound A (comparative example) or B (example 2).
FIG. 2 shows the tumor cell viability after exposing the CAL51 cell line (derived from breast carcinoma cells) during 4 hours to 20, 40 or 60µM concentrations of compound A (comparative example) or B (example 2).

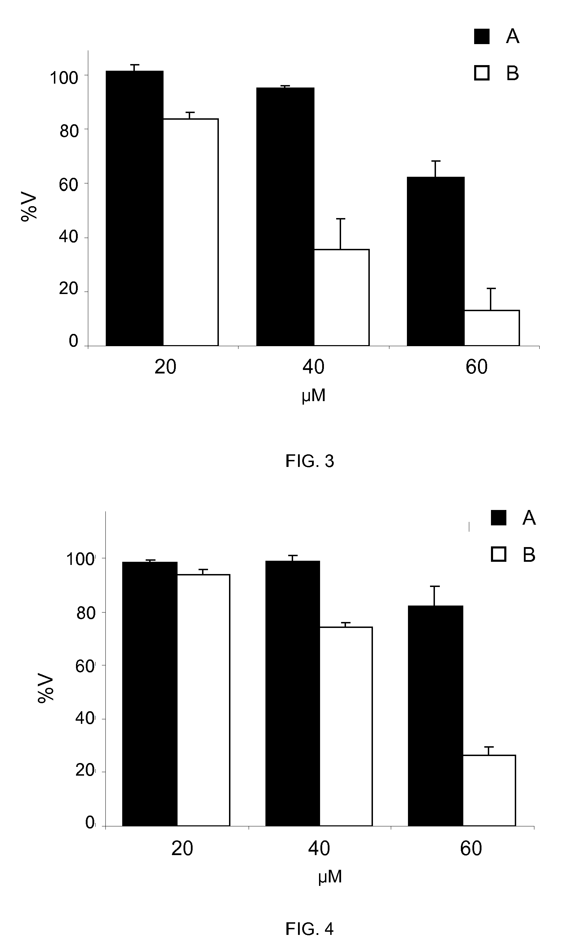

FIG. 3 shows the tumor cell viability after exposing the H-460 cell line (derived from human lung carcinoma cells) during 4 hours to 20, 40 or 60µM concentrations of compound A (comparative example) or B (example 2).
FIG. 4 shows the tumor cell viability after exposing the H-727 cell line (derived from human lung carcinoma cells) during 4 hours to 20, 40 or 60µM concentrations of compound A (comparative example) or B (example 2).

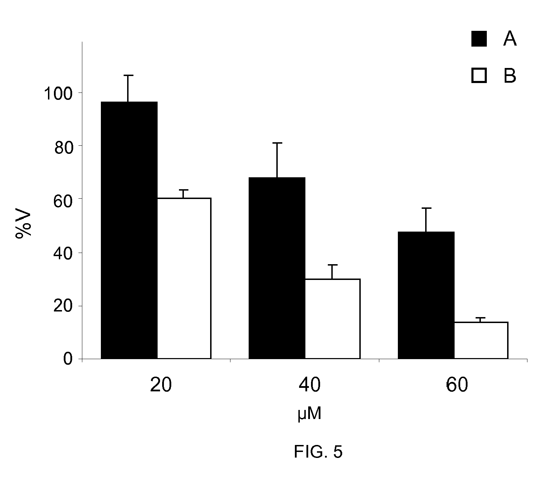

FIG. 5 shows the tumor cell viability after exposing the PC3 cell line (derived from human prostate carcinoma cells) during 4 hours to 20, 40 or 60µM concentrations of compound A (comparative example) or B (example 2).

### DETAILED DESCRIPTION OF THE INVENTION

According to the invention, a first aspect relates to compounds of formula (I) as defined above, as well as to their pharmaceutically acceptable salts or pharmaceutically acceptable solvates.

For the purposes of this invention, the term (C₁-C₄)alkyl means a straight or branched alkyl chain which contains from 1 to 4 carbon atoms and which can be optionally substituted unless otherwise stated. Examples include, among others, the groups methyl, ethyl, propyl, isopropyl, butyl, isobutyl, sec-butyl and tert-butyl.

The term halogen means fluoro, chloro, bromo or iodo.

The expression "optionally substituted with one or more" means that a group can be substituted with one or more, preferably with 1, 2, 3 or 4 substituents, provided that this group has 1, 2, 3 or 4 positions susceptible of being substituted.

There is no limitation on the type of salt that can be used, provided that these are pharmaceutically acceptable when they are used for therapeutic purposes. The term "pharmaceutically acceptable salts", embraces salts commonly used to form alkali metal salts and to form addition salts of free acids or free bases.

Salts can be prepared by treating the compound of formula (I) with a sufficient amount of the desired acid or base to give a salt in the conventional manner. The compounds of formula (I) and their salts may differ in some physical properties but they are equivalent for the purposes of the present invention.

Some of the compounds of the present invention can exist in solvated form, including hydrated forms. In general, the solvated forms with pharmaceutically acceptable solvents such as water, ethanol and the like are equivalent to the unsolvated form for the purposes of the invention.

In a preferred embodiment of the invention, in a compound of formula (I), R³ represents H.

R¹ and R² may be placed at any of the available positions of the phenyl ring. In a preferred embodiment, R¹ and R² are placed either at the positions 2 and 5 of the phenyl ring; at the positions 3 and 5 of the phenyl ring; or at the positions 2 and 3. In a more preferred embodiment, R¹ and R² are placed either at the positions 2 and 5 of the phenyl ring; or at the positions 3 and 5 of the phenyl ring.

In a more preferred embodiment, R³ represents H, and R¹ and R² are placed either at the positions 2 and 5 of the phenyl ring, or at the positions 3 and 5 of the phenyl ring.

In another preferred embodiment, R¹ and R² are individually selected from the group consisting of halogen and (C₁-C₄)alkyl optionally substituted with one or more substituents selected from the group consisting of halogen, OH and O(C₁-C₄)alkyl. In a more preferred embodiment, R¹ and R² are individually selected from the group consisting of halogen and (C₁-C₄)alkyl optionally substituted with one or more halogen atoms. In an even more preferred embodiment, R¹ and R² are individually selected from the group consisting of chloro, bromo, fluoro, methyl and trifluoromethyl.

In another preferred embodiment, R¹ and R² have the same meaning. In a more preferred embodiment, R³ represents H, and R¹ and R² have the same meaning and are placed either at the positions 2 and 5 of the phenyl ring, or at the positions 3 and 5 of the phenyl ring respectively; and are individually selected from the group consisting of halogen and (C₁-C₄)alkyl optionally substituted with one or more halogen atoms.

In another preferred embodiment of the invention the compound of formula (I) is selected from the group consisting of:
4-[2-(2,5-Dimethylphenyl)-4-(trifluoromethyl)-1H-pyrrol-1-yl]benzene-sulfonamide,
4-[2-(3,5-Bis(trifluoromethyl)phenyl)-4-(trifluoromethyl)-1H-pyrrol-1-yl]benzenesulfonamide,
4-[2-(3,5-Dimethylphenyl)-4-(trifluoromethyl)-1H-pyrrol-1-yl]benzene-sulfonamide,
4-[2-(3,5-Dichlorophenyl)-4-(trifluoromethyl)-1H-pyrrol-1-yl]benzene-sulfonamide, and
4-[2-(2,5-Dichlorophenyl)-4-(trifluoromethyl)-1H-pyrrol-1-yl]benzene-sulfonamide.

The compounds of formula (I) as defined above may be obtained in general by coupling of a compound of formula (II) with a compound of formula (III) as shown in the following scheme: 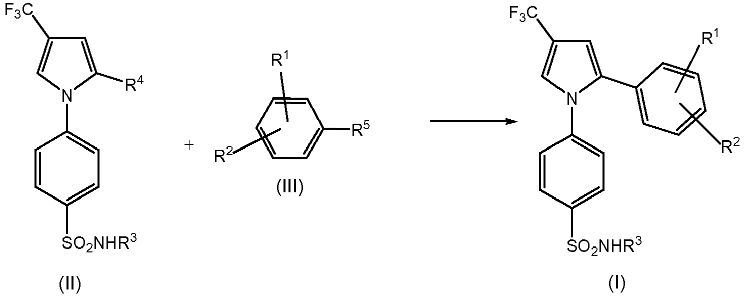 wherein herein R¹, R², R³, R⁴ and R⁵ have the same meaning as previously described.

In a preferred embodiment, R⁴ represents X and R⁵ represents Y. In a more preferred embodiment, Y is selected from Sn(CH₃)₃, ClZnMg, ClZnCu, B(OH)₂, B(OR)₂ and 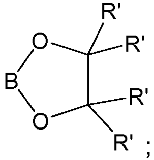 wherein R and R' have the meaning previously described.

In a more preferred embodiment, R⁴ is selected from halogen, trifluoromethanesulfonate and trimethylsilyloxy, and R⁵ is selected from B(OH)₂, B(OR)₂ and 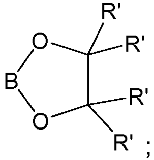 wherein R and R' have the same meaning as previously described. This embodiment has the advantage that the intermediate compounds are more easily available. In a more preferred embodiment, R⁴ represents trifluoromethanesulfonate and Y represents B(OH)₂.

In each particular case, the best reaction conditions to carry out this process (temperature, solvent and the like) may vary depending on the starting materials used, and can be easily determined by a person skilled in the art.

Generally, this reaction may be carried out in the presence of a suitable metal catalyst and a base in a suitable solvent system, at a temperature comprised between room temperature and the temperature of the boiling point of the solvent used. Preferably, the reaction is carried out at reflux.

Preferably, the metal catalyst is selected from the group consisting of a palladium or a nickel compound. More preferably the metal catalyst is selected from Tetrakis(triphenylphosphine)palladium(0) (Pd(PPh₃)₄), [1,1'-Bis(diphenylphosphino)ferrocene]dichloropalladium(II) (PdCl₂(dppf)) and palladium(II) acetate/triphenylphosphine (Pd(OAc)₂/PPh₃).

The solvent system used is preferably selected from water or an organic solvent such as a polar aprotic solvent, or mixtures thereof. Examples of such solvents include, but are not limited to, dimethylformamide (DMF), tetrahydrofuran (THF) or 1,4-dioxane. More preferably, the solvent is 1,4-dioxane.

The base is preferably selected from an amine, a carbonate and a phosphate of an alkaline metal. More preferably, the base is selected among triethylamine, sodium carbonate, potassium carbonate and potassium phosphate.

A compound of formula (I) may also be converted into another compound of formula (I), in one or a plurality of steps.

Thus, for example, a compound of formula (I) wherein R¹ or R² is (C₁-C₄)alkyl substituted with OH can be converted into a compound of formula (I) wherein R¹ or R² is (C₁-C₄)alkyl substituted with O(C₁-C₄)alkyl by an alkylation reaction well known in the art.

Or, a compound of formula (I) wherein R³ is SO₂NH₂ can be converted into a compound of formula (I) wherein R³ is SO₂NH(C₁-C₄)alkyl by an alkylation reaction well known in the art.

Or, a compound of formula (I) wherein R³ is SO₂NH₂ can be converted into a compound of formula (I) wherein R³ is SO₂NHCONH₂ or SO₂NH(C=NH)NH₂ by a transamidation reaction in the presence of urea or guanidine respectively, in an appropriate solvent, such as cyclohexanol, and preferably by heating.

Further, when in a compound of formula (II), R⁴ represents X, i.e. compound of formula (IIa), these compounds can be conveniently prepared following the synthetic process show in the following scheme: 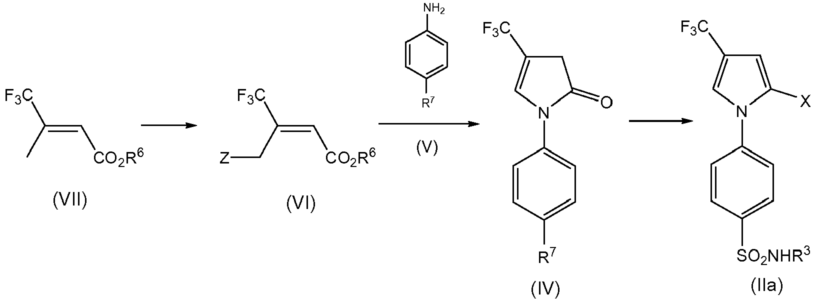 wherein R³ and X have the meaning previously described; R⁶ represents (C₁-C₆)alkyl; Z represents halogen, preferably Br; and R⁷ represents H or SO₂NHR³.

According to this process, a compound of formula (VII), which can be obtained from the corresponding carboxylic acid by a conventional synthesis, can be halogenated to give rise to a compound of formula (VI). This reaction may be carried out with N-bromosuccinimide (NBS), in the presence of a radical initiator, such as 2,2'-azobis(isobutyronitrile) (AIBN), benzoyl peroxide or meta-chloroperbenzoic acid, in a suitable solvent, such as CCl₄, and optionally using a 200 Watt lamp for initiation. Alternatively, photochemical agents may be also used as radical initiators.

The compound of formula (VI) is subsequently reacted with a compound of formula (V) to yield a lactam of formula (IV). This later reaction takes place in the presence of a base, such as 2,3,5-trimethylpyridine (TMP), in a suitable solvent, such as dimethylformamide (DMF). Other bases like colidine or triethylamine (TEA), and other polar non protic solvents, such as dimethylsulfoxide (DMSO) or diethilenglycol (DEG) can also be used.

When in the compound of formula (V) R⁷ represents SO₂NHR³, the lactam (IV) is converted into the pyrrole of formula (IIa) without any further steps. However, when in the compound of formula (V) R⁷ represents H, the resulting lactam (IV) is converted into another lactam of formula (IV) wherein R⁷ represents SO₂NHR³. This reaction may be carried out in the presence of a compound of formula ZSO₂NHR³, wherein Z represents halogen, preferably Cl, in a suitable solvent, such as chloroform, CCl₄ or dichloromethane.

The lactam (IV) wherein R⁷ represents SO₂NHR³ is further converted into the pyrrole of formula (IIa). When in a compound of formula (IIa), X represents halogen, the reaction is carried out by reacting the lactam (IV) with POCl₃ or POBr₃, preferably at reflux temperature for several hours.

When in a compound of formula (IIa), X represents trifluoromethanesulfonate, the reaction is carried out by reacting the lactam (IV) with trifluoromethanesulfonic anhydride, preferably at low temperature in a suitable solvent, such as dichloromethane (DCM).

When in a compound of formula (IIa), X represents R₃SiO, wherein R is (C₁-C₄)alkyl, the reaction may be carried out by reacting the lactam (IV) with R₃SiZ, wherein Z is halogen, preferably iodo, preferably at low temperature in a suitable solvent, such as dichloromethane (DCM).

A compound of formula (II) wherein R⁴ represents a radical from a boronic or a stannane derivative can be conveniently prepared by reacting a compound of formula (IIa) wherein X represents halogen, preferably Br, with an organometallic compound, such as butyl lithium (BuLi) or lithium diisopropylamide (LDA) at low temperature. Generally, this reaction is carried out in a suitable polar aprotic solvent, such as diethyl ether or tetrahydrofuran (THF), and at low temperatures, preferably at -75°C.

The obtained lithium intermediate can be subsequently reacted with a boron derivative of formula B(OR)₃, wherein R is (C₁-C₄)alkyl, to obtain a compound of formula (II) wherein Y is B(OH)₂. This reaction may be carried out in diethyl ether or tetrahydrofuran (THF) at temperatures between -78°C and room temperature.

If desired, the compound of formula (IIb') can be converted into another compound of formula (II) wherein Y is B(OR)₂, by reacting the compound of formula (II) wherein Y is B(OH)₂ with an alcohol of formula ROH. This reaction may be carried out in diethyl ether or tetrahydrofuran (THF) at temperatures between -78°C and room temperature.

Or, if desired the compound of formula (II) wherein Y is B(OH)₂ can be converted into another compound of formula (II) wherein Y is 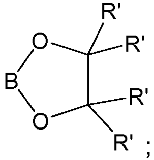 by reacting the compound of formula (II) wherein Y is B(OH)₂ with a diol of formula HOC(R'R')-C(R'R')OH.

The above obtained lithium intermediate can also be subsequently reacted with a stannane derivative of formula ZSnR₃, wherein Z is halogen, preferably bromo. This reaction may be carried out in diethyl ether or tetrahydrofuran (THF) at temperatures between -78°C and room temperature.

A compound of formula (II) wherein R⁴ represents ZZnMg, Z being preferably Cl, can be conveniently prepared by reacting a compound of formula (IIa) wherein X represents halogen, preferably Br, with an organometallic compound, such as methylmagnesium iodide or metallic magnesium to give a organomagnesian intermediate, and subsequently adding a zinc derivative, such as ZnCl₂.

A compound of formula (II) wherein R⁴ represents ZZnCu, Z being preferably Cl, can be conveniently prepared by reacting the organomagnesian intermediate as obtained above with ClCu, wherein Z is halogen, preferably Cl, and subsequently adding a zinc derivative, such as ZnCl₂.

Generally, all these reactions for preparing the magnesian or cuprate derivatives are carried out in a suitable solvent, such as diethyl ether, tetrahydrofuran (THF) or dioxane, and preferably at low temperatures. If heating is necessary, diethylenglycol as solvent may be used.

The compounds of formulas (V) and (VII) are commercially available or can be obtained by conventional synthetic processes.

The present invention also relates to a pharmaceutical composition comprising a compound of formula (I) together with excipients or other auxiliary agents if necessary. The election of the pharmaceutical formulation will depend upon the nature of the active compound and its route of administration. Any route of administration may be used, for example oral, parenteral and topical administration.

For example, the pharmaceutical composition may be formulated for oral administration and may contain one or more physiologically compatible carriers or excipients, in solid or liquid form. These preparations may contain conventional ingredients such as binding agents, fillers, lubricants, and acceptable wetting agents.

The pharmaceutical composition may be formulated for parenteral administration in combination with conventional injectable liquid carriers, such as water or suitable alcohols. Conventional pharmaceutical excipients for injection, such as stabilizing agents, solubilizing agents, and buffers, may be included in such compositions. These pharmaceutical compositions may preferably be injected intramuscularly, intraperitoneally, or intravenously.

The pharmaceutical compositions may be in any form, including, among others, tablets, pellets, capsules, aqueous or oily solutions, suspensions, emulsions, or dry powdered forms suitable for reconstitution with water or other suitable liquid medium before use, for immediate or retarded release.

The specific dose of the compound of the invention to obtain a therapeutic benefit may vary depending on the particular circumstances of the individual patient including, among others, the size, weight, age and sex of the patient, the nature and stage of the disease, the aggressiveness of the disease, and the route of administration. For example, a daily dosage of from about 0.01 to about 100 mg/kg/day may be used.

The compounds of the present invention are useful in the treatment of cancer. In a preferred embodiment, the cancer is selected from the group consisting of lung carcinoma, colorectal carcinoma, breast carcinoma and prostate carcinoma.

The compounds of the present invention, in addition to the higher antitumor activity as compared to the closest compounds of the prior art, do not show COX-2 inhibition. COX-2 inhibitors have been associated with some undesired effects, in particular with cardiovascular toxicity.

### EXAMPLES

The following examples are provided for illustrative means, and are not meant to be limiting of the present invention.

### Compound of formula (II): 1-(4-Sulfamoylphenyl)-4-(trifluoromethyl)-1H-pyrrol-2-yl trifluoromethanesulfonate (R² = trifluoromethanesulfonate, R³ = NH₂)

### a) 4-(2-Oxo-4-(trifluoromethyl)-2,3-dihydro-1H-pyrrol-1-yl)benzenesulfonamide (IV, R³ = NH₂)

A mixture of 4-aminophenylsulfonamide (V, R³ = NH₂) (0.77 mmol) and 2,3,5-trimethylpyridine (0.1 mL, 0.78 mmol) in dry dimethylformamide (DMF) (3 mL) was added to a cold (0 °C) solution of ethyl 4-bromo-3-(trifluoromethyl)-2-butenoate (VI, Z = Br, R⁶ = CH₂CH₃, obtained as in S. C. Welch, J. M. Gruber, J. Org. Chem. 1982, vol. 47, p. 385) (0.2 g, 0.77 mmol) in dry DMF (3 mL). When the addition was finished, the cooling bath was removed and the reaction mixture was stirred at room temperature for 24 hours. After this time, H₂O was added and the reaction mixture extracted with ethyl acetate (EtOAc). The organic solution was washed with 1 M HCl (2 x 5 mL) and brine (2 x 5 mL), dried with MgSO₄ anhydrous. The solvent was removed and the residue purified by column cromatography using silica gel. Elution with hexane/EtOAc (1:1) gave 11 % yield. M.p. 209-211°C. IR (KBr): 3300, 3297, 1703, 1245, 1159, 883, 607. ¹H NMR (200 MHz, (CD₃)₂CO) δ 2.84 (s, 2H); 6.58 (bs, 2H); 6.87 (q, J = 1.8 Hz, 1 H H5); 7.91 (d, J = 9.0 Hz, 2H); 8.05 (d, J = 9.0 Hz, 2H). ¹³C NMR (100 MHz, (CD₃)₂CO) δ 50.2 (t); 118.5 (d); 127.4 (d); 130.3 (d); 130.4 (s); 139.8 (s); 142.0 (s); 167.2 (s). MS (EI) 307 (M+1, 16); 306 (M, 100); 226 (50); 198 (53).

### b) Title compound

A cold (0 °C) solution of pyrrolone IV (R³ = NH₂) (8.85 mmol), as obtained above, in dry dichloromethane (DCM) (17 mL) was slowly added to trifluoromethanosulphonic anhydride (4 eq., 0.57 mL, 3.4 mmol). The reaction mixture was stirred at 0-5 °C for 30 min. The cooling bath was removed and the mixture was stirred at room temperature for 21 h. After this time DCM was added. The organic solution was washed with water (3 x 15 mL) and dried with MgSO₄. The solvent was removed and the reaction crude was purified by colum chromatography with silica gel using hexane/EtOAc as eluent. Yield 37% M.p. 118-119 °C. IR (KBr): 3360, 3280, 3220; 1526, 1430, 1160, 1127, 839. ¹H NMR (100 MHz, CDCl₃) δ 6.41 (s, 1 H); 7.08 (s, 1 H); 7.55 (d, *J* = 8.8 H, 2H); 8.10 (d, *J* = 8.8 H, 2H). ¹³C NMR (100 MHz, CDCl₃): 97.5 (d); 117.7 (d); 125.7 (d); 128.4 (d); 138.9 (s); 142.5 (s); 147.5 (s). MS (EI) 439 (M+1, 0,1); 438 (M, 5); 305 (100);

### Compounds of formula (I): General process

A solution of a compound of formula (II) (R² = trifluoromethanesulfonate, R³ = NH₂) (1 eq.), as obtained above, a compound of formula (III) (R⁵ = B(OH)₂) (4 eq.), K₂CO₃ (8 eq.) in 1,4-dioxane (10 mL) was stirred under nitrogen at room temperature for 25 min and Pd(PPh₃)₄ (0,1 eq.) was added. The reaction mixture was stirred at reflux temperature for 1 h. After this time the reaction was cooled and water (15 mL) was added. The aqueous mixture was extracted with EtOAc (3 x 10 mL), washed with 1 N NaOH (2 x 10 mL), with saturated Na₂CO₃ (2 x 5 mL) and finally with water (2 x 10 mL). The organic solution was dried and evaporated. The crude was purified by silica gel column chromatography.

Following this general process and using the suitable compound of formula (III) in each case, the following compounds were obtained:

### Example 1: 4-[2-(2,5-Dimethylphenyl)-4-(trifluoromethyl)-1H-pyrrol-1-yl]benzenesulfonamide (R¹, R² = 2-CH₃, 5-CH₃, R³ = H)

Starting compound of formula (III): (2,5-Dimethylphenyl)boronic acid (R¹, R² = 2-CH₃, 5-CH₃, R⁵ = B(OH)₂).
Yield 21%. ¹H NMR (400 MHz, CDCl₃) δ 2.27 (s, 3H); 2.33 (s, 3H); 6.46 (s, 1 H); 7.02-7.06 (m, 3H); 7.20 (d, *J* = 8.8 Hz, 2H); 7.29 (s, 1 H); 7.82 (d, *J* = 8.8 Hz, 2H). MS (Cl, NH₃): 412 (M+NH₃, 100); 395 (M+1, 74); 394 (M, 40); 375 (17). ¹³C NMR (100 MHz, CDCl₃) δ 19.8 (q); 21.1 (q); 121.2 (d); 122.2 (d); 124.9 (d); 127.9 (d); 130.1 (d); 130.6 (d); 132.1 (d); 134.5 (s);135.8 (s); 143.3 (s); 156.8 (s).

### Example 2: 4-[2-(3,5-Bis(trifluoromethyl)phenyl)-4-(trifluoromethyl)-1H-pyrrol-1-yl]benzenesulfonamide (R¹, R² = 3-CF₃, 5-CF₃, R³ = H).

Starting compound of formula (III): [3,5-Bis(trifluoromethyl)phenyl]boronic acid (R¹, R² = 3-CF₃, 5-CF₃, R⁵ = B(OH)₂).
Yield 42%. IR (KBr): 3300, 3277, 1577, 1501, 1367, 1280, 1184. ¹H NMR (CDCl₃, 400 MHz) δ 4.89 (s, 2H); 6.81 (s, 1 H); 7.28 (d, *J* = 8.4 Hz, 2H); 7.31 (s, 1 H); 7.33 (s, 2H); 7.68 (s, 1 H); 8.00 (d, *J* = 8.4 Hz, 2H). ¹³C NMR (100 MHz, CDCl₃) δ 110.1 (d); 121.1 (d); 124.2 (s); 126.4 (d); 128.1 (d); 131.8 (s); 141.9 (s); 142.1 (d). MS (Cl, NH₃) 520 (M+NH₃, 100); 502 (M, 27).

### Example 3: 4-[2-(3,5-Dimethylphenyl)-4-(trifluoromethyl)-1H-pyrrol-1-yl]benzenesulfonamide (R¹, R² = 3-CH₃, 5-CH₃, R³ = H).

Starting compound of formula (III): (3,5-Dimethylphenyl)boronic acid (R¹, R² = 3-CH₃, 5-CH₃, R⁵ = B(OH)₂).
Yield 10%. IR (KBr): 3300, 2268, 1600, 1346 1259, 1164, 1113. ¹H NMR (400 MHz, CDCl₃) δ 2.21 (s, 3H); 2.44 (s, 3H);4.89 (bs, 2H); 6.72 (s, 1 H); 6.91 (s, 1 H); 7.29 (d, *J* = 8.4 Hz, 2H); 7. 90 (d, *J* = 8.4 Hz, 2H). ¹³C NMR (100 MHz, CDCl₃) δ 21.2 (q); 21.3 (q); 108.4 (d); 122.3 (d); 125.9 (d); 126.6 (d); 127.7 (d); 129.6 (d); 130.9 (s); 131.2 (s); 133.3 (s); 134.4 (s); 137.3 (s);138.1 (s); 140.5 (s). MS (EI): 396 (M+2, 26); 395 (M+1, 38); 394 (M, 100).

### Example 4: 4-[2-(3,5-Dichlorophenyl)-4-(trifluoromethyl)-1H-pyrrol-1-yl]benzenesulfonamide (R¹, R² = 3-Cl, 5-Cl, R³ = H).

Starting compound of formula (III): (3,5-Dichlorophenyl)boronic acid (R¹, R² = 3-Cl, 5-Cl, R⁵ = B(OH)₂).
Yield 15%. IR (KBr): 3300, 3271, 1597, 1560, 1262, 1165. ¹H NMR (CDCl₃, 400 MHz) δ 6.66 (s, 1 H); 6.98 (s, 2H); 7.31 (d, *J* = 8.4 Hz, 2H); 7.98 (d, *J* = 8.4 Hz, 2H). ¹³C NMR (CDCl₃, 100 MHz) δ 109.9 (d); 123.7 (d); 125.9 (d); 126.7 (d); 127.8 (d); 128.1 (d); 131.9 (s); 132.2 (s); 133.8 (s); 135.2 (s); 141.5 (s); 142.1 (s). MS (EI): 436 (M+1, 67); 435 (M, 28); 434 (100).

### Example 5: 4-[2-(2,5-Dichlorophenyl)-4-(trifluoromethyl)-1H-pyrrol-1-yl]benzenesulfonamide (R¹ = 2-Cl, R² = 5-Cl, R³ = H).

Starting compound of formula (III): (2,5-Dichlorophenyl)boronic acid (R¹, R² = 2-Cl, 5-Cl, R⁵ = B(OH)₂).
Yield 20%. HPLC-MS: 435 (M⁺), 871 (2M⁺ + H). ¹H NMR (CDCl₃, 400 MHz) δ 4.84 (s, 2H); 6.60 (s, 1 H); 7.23 (s, 1 H); 7.24 (d, *J* = 8.8 Hz, 2H); 7.27 (d, *J* = 2.4 Hz, 1 H); 7.32 (m, 1 H); 7.36 (d, *J* = 2.4 Hz, 1 H); 7.88 (d, *J* = 8.8 Hz, 2H). ¹³C NMR (CDCl₃, 100 MHz) δ 110.5 (d); 122.2 (d); 124.9 (d); 127.9 (d); 128.7 (s); 130.3 (d); 130.5 (s); 131.1 (d); 132.0 (s); 132.3 (d); 132.8 (s); 140.8 (s); 142.6 (s); 149.9 (s); 153.4 (s).

Following the general process described above but using (4-fluorophenyl)-boronic acid instead of a compound of formula (III), the following comparative example was obtained:

### Comparative example: 4-[2-(4-Fluorophenyl)-4-(trifluoromethyl)-1H-pyrrol-1-yl]benzenesulfonamide.

Yield 14,3%. HPLC-MS: 385 (M⁺+H), 408 (M⁺ +Na+H), 769 (2M⁺+H). ¹H NMR (CDCl₃, 400 MHz) δ 4.82 (s, 2H); 6.57 (s, 1 H); 6.98 - 7.11 (m, 5H); 7.28 (d, *J* = 8.7 Hz, 2H); 7.92 (d, *J* = 8.7 Hz, 2H).

### Antitumor activity

### Cell lines and culture conditions

H-727 and H-460 cell lines, derived from human lung carcinoma, and human prostate carcinoma PC3 cells, were cultured in RPMI 1640 medium supplemented with 10% fetal bovine serum, 1% glutamine and 100 units/ml penicillin/streptomycin (Life Technologies, Inc.). LS174T cells, derived from human colorectal carcinoma, and CAL51 cells, from breast carcinoma, were cultured in DMEM medium supplemented with 10% fetal bovine serum, 1% glutamine and 100 units/ml penicillin/streptomycin (Life Technologies, Inc.). All cell lines were incubated at 37°C in a humidified atmosphere containing 5% CO₂. The stock solution of each compound was reconstituted in DMSO and diluted in culture media before use.

### Cell viability assays

Antitumor activity was evaluated measuring cell metabolic capacity (viability), using the Cell Proliferation Kit II (XTT) and following the recommendations of the manufacturer (Roche Diagnostics). The assays were carried out in triplicates, with controls containing unexposed cells, cells with vehicle, or media plus compound. Cells were seeded into 96-well plates in 100 µl of media and incubated for 24 h. Afterwards, the compound of Example 2, the compound of the comparative example or the vehicle were added at 20, 40 or 60µM concentrations, and incubated for 4h. At the end of the incubation period, 50 µl of a mixture containing XTT and electron coupling reagent were added to each well. After 4h of incubation at 37°C, the absorbance at 490 nm was read. The growth inhibitory activity was obtained subtracting the absorbance of the blanks and expressed as percentage of cell growth inhibition, as compared with untreated controls.

As shown in the figures, the compound of example 2 showed a significantly reduced tumor cell viability in all these cancer cell lines in comparison with the comparative compound of the prior art. In particular, the compound of the invention showed a reduction in the tumor cell viability percentage of more than 60% at 40 µm as compared to the comparative example in the LS174T, CAL51 and H-460 cell lines. And the reduction at 40 µm was more than 50% in the LS174T, CAL51, H-460 and H-727 cell lines.

## Claims

1. A compound of formula (I), or a pharmaceutically acceptable salt thereof, or a pharmaceutically acceptable solvate thereof, including a hydrate, 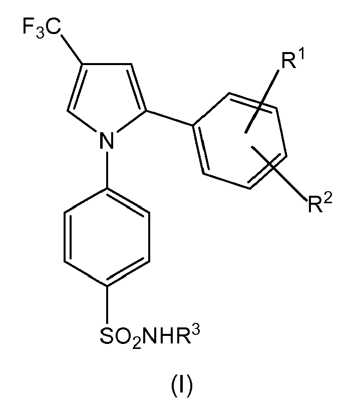 wherein
R¹ and R² are individually selected from the group consisting of halogen, O(C₁-C₄)alkyl optionally substituted with one or more halogen atom, and (C₁-C₄)alkyl optionally substituted with one or more substituents selected from the group consisting of halogen, OH and O(C₁-C₄)alkyl; and
R³ is selected from the group consisting of H, (C₁-C₄)alkyl, CONH₂ and (C=NH)NH₂.

2. The compound according to claim 1, wherein R³ represents H.

3. The compound according to any of the claims 1-2, wherein R¹ and R² are placed at the positions 2 and 5 of the phenyl ring.

4. The compound according to any of the claims 1-2, wherein R¹ and R² are placed at the positions 3 and 5 of the phenyl ring.

5. The compound according to any of the claims 1-4, wherein R¹ and R² are individually selected from the group consisting of halogen and (C₁-C₄)alkyl optionally substituted with one or more substituents selected from the group consisting of halogen, OH and O(C₁-C₄)alkyl.

6. The compound according to claim 5, wherein R¹ and R² are individually selected from the group consisting of halogen and (C₁-C₄)alkyl optionally substituted with one or more halogen atoms.

7. The compound according to claim 6, wherein R¹ and R² are individually selected from the group consisting of chloro, bromo, fluoro, methyl and trifluoromethyl.

8. The compound according to any of the claims 1-7, wherein R¹ and R² have the same meaning.

9. The compound according to any of the claims 1-8, selected from the group consisting of:
4-[2-(2,5-Dimethylphenyl)-4-(trifluoromethyl)-1H-pyrrol-1-yl]benzene-sulfonamide,
4-[2-(3,5-Bis(trifluoromethyl)phenyl)-4-(trifluoromethyl)-1H-pyrrol-1-yl]benzenesulfonamide,
4-[2-(3,5-Dimethylphenyl)-4-(trifluoromethyl)-1H-pyrrol-1-yl]benzene-sulfonamide,
4-[2-(3,5-Dichlorophenyl)-4-(trifluoromethyl)-1H-pyrrol-1-yl]benzene-sulfonamide, and
4-[2-(2,5-Dichlorophenyl)-4-(trifluoromethyl)-1H-pyrrol-1-yl]benzene-sulfonamide.

10. A process for preparing a compound of formula (I) according to claim 1, which comprises:
a) reacting a compound of formula (II) with a compound of formula (III) 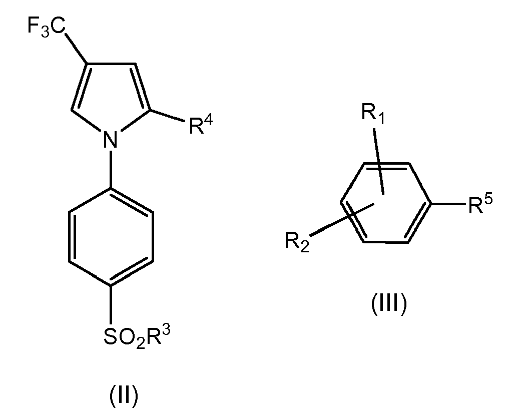 in the presence of a metal catalyst and a base, wherein
R¹, R² and R³ have the same meaning as defined in claim 1;
R⁴ represents X or Y;
R⁵ represents X when R⁴ represents Y, or R⁵ represents Y when R⁴ represents X;
X is selected from the group consisting of halogen, trifluoromethanesulfonate and R₃SiO;
Y is selected from the group consisting of SnR₃, ZZnMg, ZZnCu, B(OH)₂, B(OR)₂ and 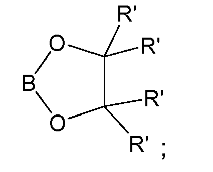
R represents (C₁-C₄)alkyl;
Z represents halogen; and
R' represents H or (C₁-C₄)alkyl;
b) optionally converting, in one or a plurality of steps, a compound of formula (I) into another compound of formula (I); and
c) optionally reacting a compound of formula (I) with a base or an acid to give the corresponding salt.

11. The process according to claim 10, wherein R⁴ represents X and R⁵ represents Y.

12. The process according to claim 11, wherein R⁴ represents trifluoromethanesulfonate and R⁵ represents B(OH)₂.

13. A pharmaceutical composition which comprises an effective amount of a compound of formula (I) according to any of the claims 1-9, or a pharmaceutically acceptable salt or a pharmaceutically acceptable solvate thereof, together with one or more pharmaceutically acceptable excipients or carriers.

14. A compound of formula (I) according to any of the claims 1-9, or a pharmaceutically acceptable salt or solvate thereof, for use in the treatment of cancer.

15. The compound for use of formula (I) according to claim 14, wherein the cancer is selected from the group consisting of lung carcinoma, colorectal carcinoma, breast carcinoma and prostate carcinoma.

## Patentansprüche

1. Verbindung der Formel (I), oder ein pharmazeutisch akzeptables Salz davon, oder ein pharmazeutisch akzeptables Solvat davon, umfassend ein Hydrat, 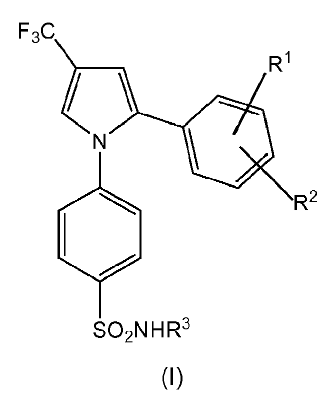 worin
R¹ und R² individuell ausgewählt sind aus der Gruppe bestehend aus Halogen, O(C₁-C₄)-Alkyl, wahlweise mit einem oder mehreren Halogenatomen substituiert, und (C₁-C₄)-Alkyl, wahlweise mit einem oder mehreren Substituenten substituiert, die aus der Gruppe bestehend aus Halogen, OH und O(C₁-C₄)-Alkyl ausgewählt sind; und
R³ ausgewählt ist aus der Gruppe bestehend aus H, (C₁-C₄)-Alkyl, CONH₂ und (C=NH)NH₂.

2. Verbindung nach Anspruch 1, wobei R³ für H steht.

3. Verbindung nach einem der Ansprüche 1-2, wobei R¹ und R² sich an den Positionen 2 und 5 des Phenylrings befinden.

4. Verbindung nach einem der Ansprüche 1-2, wobei R¹ und R² sich an den Positionen 3 und 5 des Phenylrings befinden.

5. Verbindungen nach einem der Ansprüche 1-4, wobei R¹ und R² individuell ausgewählt sind aus der Gruppe bestehend aus Halogen und (C₁-C₄)-Alkyl, wahlweise substituiert mit einem oder mehreren Substituenten, die aus der Gruppe bestehend aus Halogen, OH und O(C₁-C₄)-Alkyl ausgewählt sind.

6. Verbindung nach Anspruch 5, wobei R¹ und R² individuell ausgewählt sind aus der Gruppe bestehend aus Halogen und (C₁-C₄)-Alkyl, wahlweise substituiert mit einem oder mehreren Halogenatomen.

7. Verbindung nach Anspruch 6, wobei R¹ und R² individuell ausgewählt aus der Gruppe bestehend aus Chloro, Bromo, Fluoro, Methyl und Trifluoromethyl sind.

8. Verbindung nach einem der Ansprüche 1-7, wobei R¹ und R² für das Gleiche stehen.

9. Verbindung nach einem der Ansprüche 1-8, die ausgewählt ist aus der Gruppe bestehend aus:
4-[2-(2,5-Dimethylphenyl)-4-(trifluoromethyl)-1H-pyrrol-1-yl]benzolsulfonamid,
4-[2-(3,5-Bis(trifluoromethyl)phenyl)-4-(trifluoromethyl)-1H-pyrrol-1yl]benzolsulfonamid,
4-[2-(3,5-Dimethylphenyl)-4-(trifluoromethyl)-1H-pyrrol-1-yl]benzolsulfonamid,
4-[2-(3,5-Dichlorophenyl)-4-(trifluoromethyl)-1H-pyrrol-1-yl]benzolsulfonamid, und
4-[2-(2,5-Dichlorophenyl)-4-(trifluoromethyl)-1H-pyrrol-1-yl]benzolsulfonamid.

10. Verfahren zur Herstellung einer Verbindung der Formel (I) nach Anspruch 1, das folgendes umfasst:
a) die Reaktion einer Verbindung der Formel (II) mit einer Verbindung der Formel (III) 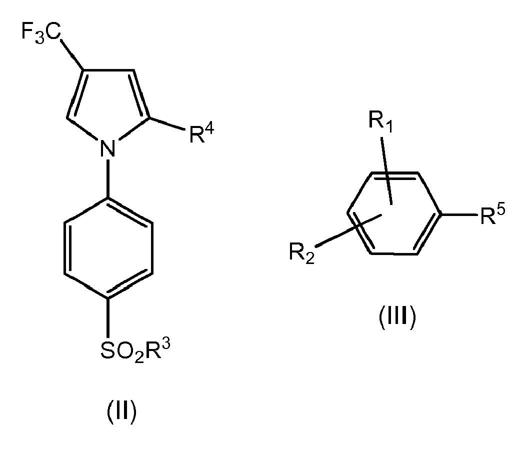 in Anwesenheit eines metallischen Katalysators und eines Alkalis, worin
R¹, R² und R³ die Bedeutung haben, die in Anspruch 1 definiert wurde;
R⁴ für X oder Y steht;
R⁵ für X steht, wenn R⁴ für Y steht, oder R⁵ für Y steht, wenn R⁴ für X steht;
X ausgewählt ist aus der Gruppe bestehend aus Halogen, Trifluoromethanosulfonat und R₃SiO;
Y ausgewählt ist aus der Gruppe bestehend aus SnR₃, ZZnMg, ZZnCu, B(OH)₂, B(OR)₂ und 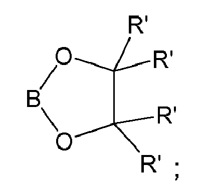
R für (C₁-C₄)-Alkyl steht;
Z für Halogen steht; und
R' für H oder (C₁-C₄)-Alkyl steht;
b) wahlweise die Umwandlung, in einem oder in mehreren Schritten, einer Verbindung der Formel (I) in eine andere Verbindung der Formel (I); und
c) wahlweise die Reaktion einer Verbindung der Formel (I) mit einem Alkali oder einer Säure, um das entsprechende Salz zu ergeben.

11. Verfahren nach Anspruch 10, wobei R⁴ für X steht und R⁵ für Y steht.

12. Verfahren nach Anspruch 11, wobei R⁴ für Trifluoromethanosulfonat steht und R⁵ für B(OH)₂ steht.

13. Pharmazeutische Zusammensetzung umfassend eine wirksame Menge an einer Verbindung der Formel (I) nach einem der Ansprüche 1-9, oder ein pharmazeutisch akzeptables Salz oder ein pharmazeutisch akzeptables Solvat davon, nebst einem oder mehreren pharmazeutisch akzeptablen Hilfsstoffen oder Arzneistoffträgern.

14. Verbindung der Formel (I) nach einem der Ansprüche 1-9, oder ein pharmazeutisch akzeptables Salz oder Solvat davon, zur Verwendung in der Behandlung von Krebs.

15. Verbindung zur Verwendung der Formel (I) nach Anspruch 14, wobei der Krebs ausgewählt ist aus der Gruppe bestehend aus Lungenkarzinom, Kolorektalkarzinom, Mammakarzinom und Prostatakarzinom.

## Revendications

1. Composé de formule (I), ou un sel pharmaceutiquement acceptable de celui-ci, ou un solvate pharmaceutiquement acceptable de celui-ci, y compris un hydrate, 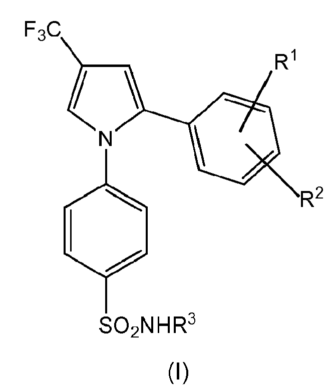 dans laquelle
R¹ et R² sont choisis individuellement dans le groupe constitué des halogènes, des -O(C₁-C₄ alkyles), facultativement substitués avec un ou plusieurs atomes d'halogène, et des alkyles en C₁-C₄ facultativement substitués avec un ou plusieurs substituants choisis dans le groupe constitué des halogènes, du groupe -OH et des -O(C₁-C₄ alkyles); et
R³ est choisi dans le groupe constitué de H, des alkyles en C₁-C₄, de CONH₂ et de (C=NH)NH₂.

2. Composé selon la revendication 1, dans lequel R³ représente H.

3. Composé selon l'une quelconque des revendications 1-2, dans lequel R¹ et R² sont placés dans les positions 2 et 5 de l'anneau phényle.

4. Composé selon l'une quelconque des revendications 1-2, dans lequel R¹ et R² sont placés dans les positions 3 et 5 de l'anneau phényle.

5. Composé selon l'une quelconque des revendications 1-4, dans lequel R¹ et R² sont choisis individuellement dans le groupe constitué des halogènes et des alkyles en C₁-C₄, facultativement substitués avec un ou plusieurs substituants choisis dans le groupe constitué des halogènes, du groupe -OH et des -O(C₁-C₄ alkyles).

6. Composé selon la revendication 5, dans lequel R¹ et R² sont individuellement choisis dans le groupe constitué des halogènes et des alkyles en C₁-C₄ facultativement substitués avec un ou plusieurs atomes d'halogène.

7. Composé selon la revendication 6, dans lequel R¹ et R² sont choisis individuellement dans le groupe constitué du chlore, du brome, du fluor, du méthyle et du trifluorométhyle.

8. Composé selon l'une quelconque des revendications 1-7, dans lequel R¹ et R² ont la même signification.

9. Composé selon l'une quelconque des revendications 1-8, choisi dans le groupe constitué de:
4-[2-(2,5-diméthylphényl)-4-(trifluorométhyl)-1H-pyrrol-1-yl]benzènesulfonamide,
4-[2-(3,5-bis(trifluorométhyl)phényl)-4-(trifluorométhyl)-1H-pyrrol-1yl]benzène-sulfonamide,
4-[2-(3,5-diméthylphényl)-4-(trifluorométhyl)-1H-pyrrol-1-yl]benzènesulfonamide,
4-[2-(3,5-dichlorophényl)-4-(trifluorométhyl)-1H-pyrrol-1-yl]benzènesulfonamide,
4-[2-(2,5-dichlorophényl)-4-(trifluorométhyl)-1 H-pyrrol-1-yl]benzènesulfonamide,

10. Procédé de préparation d'un composé de formule (I) selon la revendication 1 comprenant:
a) faire réagir un composé de formule (II) avec un composé de formule (III) 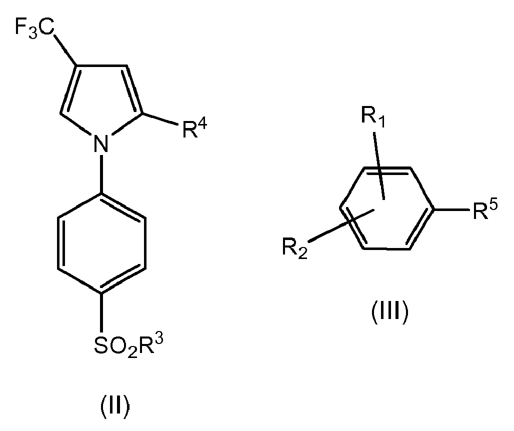 en présence d'un catalyseur métallique et d'une base, dans laquelle
R¹, R² et R³ ont la signification telle que définie dans la revendication 1;
R⁴ représente X ou Y:
R⁵ représente X lorsque R⁴ représente Y, ou R⁵ représente Y lorsque R⁴ représente X;
X est choisi dans le groupe constitué des halogènes, du trifluorométhane-sulfonate et de R₃SiO;
Y est choisi dans le groupe constitué de SnR₃, ZZnMg, ZZnCu, B(OH)₂, B(OR)₂ et 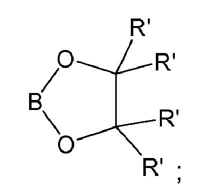
R représente un alkyle en C₁-C₄;
Z représente un halogène; et
R' représente H ou un alkyle en C₁-C₄ ;
b) facultativement transformer, dans une étape ou dans une pluralité d'étapes, un composé de formule (I) dans un autre composé de formule (I) ; et
c) facultativement faire réagir un composé de formule (I) avec une base ou un acide pour donner le sel correspondant.

11. Le procédé selon la revendication 10, dans lequel R⁴ représente X et R⁵ représente Y.

12. Procédé selon la revendication 11, dans lequel R⁴ représente le trifluoro-méthanosulfonate et R⁵ représente B(OH)₂.

13. Composition pharmaceutique qui comprend une quantité efficace d'un composé de formule (I) selon l'une quelconque des revendications 1-9, ou un sel pharmaceutiquement acceptable ou un solvate pharmaceutiquement acceptable de celui-ci, avec un ou plusieurs excipients ou véhicules pharmaceutiquement acceptables.

14. Composé de formule (I) selon l'une quelconque des revendications 1-9, ou un sel ou un solvate pharmaceutiquement acceptable de celui-ci, pour l'utilisation dans le traitement du cancer.

15. Composé pour l'utilisation de formule (I) selon la revendication 14, dans lequel le cancer est choisi dans le groupe constitué du carcinome pulmonaire, du carcinome colorectal, du carcinome du sein et du carcinome de la prostate.
